(19) Europäisches Patentamt / European Patent Office / Office européen des brevets

(11) **EP 3 083 051 B1**

(12) **EUROPEAN PATENT SPECIFICATION**

(45) Date of publication and mention
of the grant of the patent:
**28.07.2021 Bulletin 2021/30**

(21) Application number: **14802472.2**

(22) Date of filing: **25.11.2014**

(51) Int Cl.:
**B01L 3/00** (2006.01)

(86) International application number:
**PCT/EP2014/075569**

(87) International publication number:
**WO 2015/090863 (25.06.2015 Gazette 2015/25)**

(54) **METHOD FOR SEPARATION OF PARTICULATES**

VERFAHREN ZUM TRENNEN VON TEILCHEN

PROCÉDÉ DE SÉPARATION DE PARTICULES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.12.2013 US 201361916379 P
29.01.2014 US 201414167393**

(43) Date of publication of application:
**26.10.2016 Bulletin 2016/43**

(73) Proprietor: **Global Life Sciences Solutions
Operations UK Ltd
Sheffield
S9 2RX (GB)**

(72) Inventors:
• **PULEO, Christopher Michael**
**Niskayuna, New York 12309 (US)**
• **KVAM, Erik, Leeming**
**Niskayuna, New York 12309 (US)**
• **GROSSMANN, Gregory Andrew**
**Niskayuna, New York 12309 (US)**
• **GALLIGAN, Craig Patrick**
**Niskayuna, New York 12309 (US)**
• **NICHOLS, Jason Michael**
**Niskayuna, New York 12309 (US)**
• **WANG, Xuefeng**
**Niskayuna, New York 12309 (US)**
• **DAVIS, Jason Louis**
**Niskayuna, New York 12309 (US)**

(74) Representative: **Cavill, Ross David
GE Healthcare UK Limited
Pollards Wood
Nightingales Lane
Chalfont St Giles, Buckinghamshire HP8 4SP
(GB)**

(56) References cited:
**US-A1- 2005 148 064      US-A1- 2009 220 932
US-A1- 2012 149 021**

• **None**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD

The invention relates to microfluidic systems, devices and methods useful for separating particulate materials from fluids. In a particular aspect, the invention relates to a method for separating cells from biological samples using the systems, devices and methods provided herein.

BACKGROUND

Preparation and manipulation of high quality cells or biomolecules are primary requirements for a variety of diagnostic or therapeutic applications. Though filtration techniques are commonly used for capturing cells, they pose various challenges including the inability to obtain high separation efficiencies for heterogeneous cell populations, clogging of pores or harsh filtration conditions causing cellular damage. The challenges are exacerbated when filtering larger volumes of crude biological samples. In addition to filtration, a number of microfluidic separation techniques exist that rely on the application of a force, which acts to push or pull cells in a direction perpendicular to the direction of flow of the sample fluid being processed. Several of these continuous flow techniques have recently proven useful in efficiently separating out cells from blood, including hydrodynamic filtration, inertial and deterministic lateral flow separation. However, most of the techniques require pre-dilution of the blood sample and are limited to relatively low volumetric flow rates.

Recent developments on "filter-free" mechanisms for biological sample preparation offer portable purification devices for limited volumes. Primary applications have been in point-of-care diagnostics, where relatively small samples are collected and directly processed within the collection vessel. However, there remains a need to expand these simple separation methods to larger volumetric flow rates to provide alternatives to large scale centrifugation and filtration techniques.

A well-known continuous flow separation technique is field flow fractionation (FFF) in which differential retention of particles being eluted through a microchannel results in separation of particles having different characteristics. However, the relevance of field flow fractionation for whole blood separation remains uncertain as most studies suggest the need for relatively dilute starting blood samples. Recent combinations of newer separation techniques, such as hydrodynamic filtration and inertial focusing, have increased the traditional throughput limits associated with continuous flow separations. However, each of these "high throughput" implementations requires carefully controlled flow rates and/or upstream sample pre-filtration or dilution.

US2012/149021 A1 describes a device for separating particulates dispersed within a base fluid and having a relative density difference compare to the bas fluid. The particulates and a portion of the base fluid traverse the microporous body under influence of an external force field, and are entered and collected in a collection chamber. A microchannel of length 1 and height h disposed between a fluid inlet and a fluid outlet is not described. US2005/148064 A1 describes a microfluidic device and method for fractionating and/or trapping selected molecules with a diffusion barrier or porous membrane. The device includes a source fluid flow channel and a target fluid flow channel. The target fluid flow channel and the source fluid flow channel meet at cross-channel area and are in fluid communication with each other. A porous membrane separates the source fluid flow channel from the target fluid flow channel in the cross-channel area. A field-force/gradient mechanism may be positioned proximate the porous membrane with or without detection/state monitoring devices.

Sedimentation-based devices may provide simpler methods of cell and/or particle separation from fluids containing them without the need for careful fluid flow control and/or excessive sample dilution. However, there exists a need to provide devices and methods that enable high speed separation of particulates such as cells and/or dispersed particles from fluids containing them without the need to augment sedimentation rates via capital intensive equipment, such as centrifuges. A fast and efficient separation and collection of particles or cells from a large sample volume without complex equipment is an unmet need. Therefore, inexpensive devices that can accelerate particle separation via sedimentation, and enable use of a large sample volume with minimal human intervention are highly desirable.

BRIEF DESCRIPTION

In one embodiment, a method for separating particulates dispersed within a base fluid and having a relative density difference compared to the base fluid, comprises: providing a separation device comprising: a microchannel of length 1 and height h disposed between a fluid inlet and a fluid outlet; a microporous body defining at least a portion of the microchannel; and a collection chamber on an opposing side of the microporous body; wherein the particulates and a portion of the base fluid traverse the microporous body under the influence of an external force field, and are entered and collected in the collection chamber; introducing a sample of unprocessed fluid comprising particulates dispersed within a base fluid into the microchannel via the fluid inlet; separating at least a portion of the particulates from the unprocessed fluid to provide a stream of processed fluid at the fluid outlet; and recovering at least a portion of the particulates initially present in the unprocessed fluid in the collection chamber; wherein the particulates and a portion of the base fluid traverse the microporous body under the influence of an external force field, and are entered and collected in the collection chamber; and wherein the microporous body operationally generates a fluid flow regime comprising a first fluid flow having a first flow rate through the microchannel and a second fluid flow having a second flow rate through the collection chamber and the second flow rate is a fraction of the first flow rate.

One embodiment of a method for separating cells dispersed within a base fluid of whole blood sample, comprises providing a separation device comprising: a microchannel of length 1 and height h disposed between a fluid inlet and a fluid outlet; a microporous body defining at least a portion of the microchannel; and a collection chamber on an opposing side of the microporous body; wherein the particulates and a portion of the base fluid traverse the microporous body under the influence of an external force field, and are entered and collected in the collection chamber; introducing the whole blood sample of unprocessed fluid comprising cells dispersed within a base fluid into the microchannel via the fluid inlet; separating at least a portion of the cells from the unprocessed fluid to provide a stream of processed fluid at the fluid outlet; and recovering at least a portion of the cells initially present in the unprocessed fluid in the collection chamber; wherein the particulates and a portion of the base fluid traverse the microporous body under the influence of an external force field, and are entered and collected in the collection chamber; and wherein the microporous body operationally generates a fluid flow regime comprising a first fluid flow having a first flow rate through the microchannel and a second fluid flow having a second flow rate through the collection chamber and the second flow rate is a fraction of the first flow rate.

DRAWINGS

These and other features, aspects, and advantages of the present invention will become better understood when the following detailed description is read with reference to the accompanying drawings in which like characters represent like parts throughout the drawings, wherein:

FIG. 1A is a schematic drawing of front view of a device suitable for use in one embodiment of the devices.

FIG. 1B is a schematic drawing of side view of a device suitable for use in one embodiment of the devices.

FIG. 1C is a schematic drawing of top view of a device suitable for use in one embodiment of the devices.

FIG. 2 is a schematic view of method steps for separating cells from whole blood using one embodiment of the devices.

FIG. 3 illustrates a method of separating particles based on density from a fluid using one embodiment of the devices.

FIG. 4 illustrates a method of separating particles based on density from a fluid using another embodiment of the devices.

FIG. 5 illustrates a method of separating particles from a fluid using one embodiment of the devices.

FIG. 6 illustrates a method of separating particles from a fluid using one embodiment of the devices.

FIGs. 7A and 7B are images taken from computational fluid dynamic (CFD) models showing fluid flow pattern through the device without microporous surface and with microporous surface, respectively.

FIGs. 8A and 8B illustrate the performance characteristics of a cell separation device showing loss of leukocytes with increasing fluid flow rate using a device without a microporous surface and with a microporous surface, respectively.

FIGs. 8C and 8D illustrate the performance characteristics of a cell separation device, showing blood sample loaded through inlet and processed fluid samples driven out from the outlet of a device without a microporous surface and a device with a microporous surface, respectively.

FIG. 9 illustrates the performance characteristics of a cell separation device showing a percentage of cells captured in a collection chamber from a blood sample loaded into one embodiment of the device (I) compared with a commercial benchmark (II).

FIG. 10 illustrates the performance characteristics of a cell separation device showing percentage recovery of captured cells from one embodiment of the device (I) relative to a commercial benchmark (II).

FIG 11 A illustrates the performance characteristics of a cell separation device showing a percentage of captured and recovered cells from a blood sample with different flow rates using one embodiment of the device.

FIG. 11 B illustrates the performance characteristics of a cell separation device showing length for varying flow rate using one embodiment of the device.

FIG. 12 illustrates one embodiment of a microfluidic separation device.

FIG. 13 illustrates one embodiment of a portion of the microfluidic separation device of FIG. 12 in an exploded perspective view.

FIG. 14 illustrates one embodiment of a portion of the microfluidic separation device of FIG. 12 in an exploded perspective view.

DETAILED DESCRIPTION

Separation of particulates dispersed within a base fluid (collectively "the unprocessed fluid"), for example separation of blood cells dispersed within blood plasma, or separation of particulate impurities dispersed in water, may be effected using various embodiments of systems, devices and methods provided by the present invention. Embodiments of the device and its device components (e.g. FIG.s 1A-1C, FIG.s 12-13) comprise a fluid inlet for introducing the unprocessed fluid into the device, a fluid outlet for removing processed fluid from the device, and a separation region comprising a microchannel disposed between the fluid inlet and the fluid outlet, a microporous body defining at least a portion of the microchannel; and a collection chamber. The particles are separated from the base fluid of the fluidic sample through the microporous body using fluidic flow and sedimentation, which is unlike a filtration device that relies entirely on physical barriers to filter particles.

To more clearly and concisely describe the subject matter of the disclosed invention, the following definitions are provided for specific terms, which are used in the following description and the appended embodiments. Throughout the specification, exemplification of specific terms should be considered as non-limiting examples.

The terms "particulate" and "particle", and their plural referents "particulates" and "particles", are used interchangeably herein and are intended to have the same meaning, particle being treated as a synonym for particulate. As used herein, the term "particles" refers to a portion of the fluidic sample loaded into the device which excludes the base fluid. The term "particles" includes without limitation cells, inorganic colloids, polymers, biopolymers, immiscible liquids, heterogenous solids, and nominally gaseous/liquid materials in a solid phase. For example, blood cells, grains of sand, oil droplets, nucleic acids, or ice crystals are all particles.

The terms "microporous body" and "microporous surface" may be used interchangeably herein and are intended to have the same meaning.

As used herein, the term "operationally generates" refers to a function of generating one or more fluid flow regimes by microporous surface during operation of the device. For example, when a fluid sample loaded into the device and flows through the microchannel for separation of the particles from the fluid, the microporous surface generates a field of flow with two fluid flow regimes under the operating conditions of the device.

As used herein, the term "fluidic sample" refers to a mixture that comprises a non-fluid component and a fluid component. The mixture may be heterogeneous or homogenous in nature. The loaded sample is interchangeably used herein with a "sample", "fluidic sample" or "fluid loaded into the device". The sample may comprise without limitation, a fluid comprising one or more particles, a fluid comprising one or more cells, water with particles, water-oil emulsion, or a fluid with impurities. For example, a sample comprises a slurry of sand and water, or a fluidic sample comprises cells and plasma. The term "fluidic sample" is used interchangeably and without limitation with the term "dispersion", "particle dispersion", or "cellular dispersion" when identifying a generic class of fluidic sample. In some embodiments, a cellular dispersion refers to a sample of cells dispersed in a fluid, for example blood cells dispersed in plasma, cells dispersed in growth media, or cells dispersed in stabilization media.

As used herein, the term "base fluid" refers to a portion of the fluidic sample which excludes the particles. For example, a whole blood sample comprises blood cells in plasma, wherein the plasma is a base fluid. For another example, an aqueous sand dispersion comprises sand in water, wherein water is a base fluid.

As used herein, the term "relative density difference" refers to a difference between the density of the particulates present in the base fluid and the density of the base fluid.

As used herein, the term "sediment" refers to particle motion induced by an applied force field. Motion or movement of particulates in a fluid in response to a force may be active or passive, and the movement is referred to herein as sedimentation. For example, in cases where the force of gravity augments the action of an externally applied electric field in inducing particulate movement. Sedimentation usually provides a simple means of separating particulates from a base fluid. In one embodiment, in an aqueous sand dispersion, the sand particles have a density greater than that of the base fluid and sediment in the direction of the gravitational field. In another embodiment, in an oil-in-water emulsion, the oil droplets have a density less than that of the base fluid and transit in the direction opposing the gravitational field. In another embodiment, in a dispersion of magnetic particles, the magnetic particles are sediment in the direction of an applied magnetic field. In another embodiment, in a dispersion of charged particles, the charged particles differentially sediment based on their polarity within an applied electric field.

The singular forms "a", "an" and "the" include plural referents unless the context clearly dictates otherwise.

Approximating language, as used herein throughout the specification and claims, may be applied to modify any quantitative representation that could permissibly vary without resulting in a change in the basic function to which it is related. Accordingly, a value modified by a term such as "about" is not to be limited to the precise value specified. In some instances, the approximating language may correspond to the precision of an instrument for measuring the value. Where necessary, ranges have been supplied, and those ranges are inclusive of all sub-ranges there between.

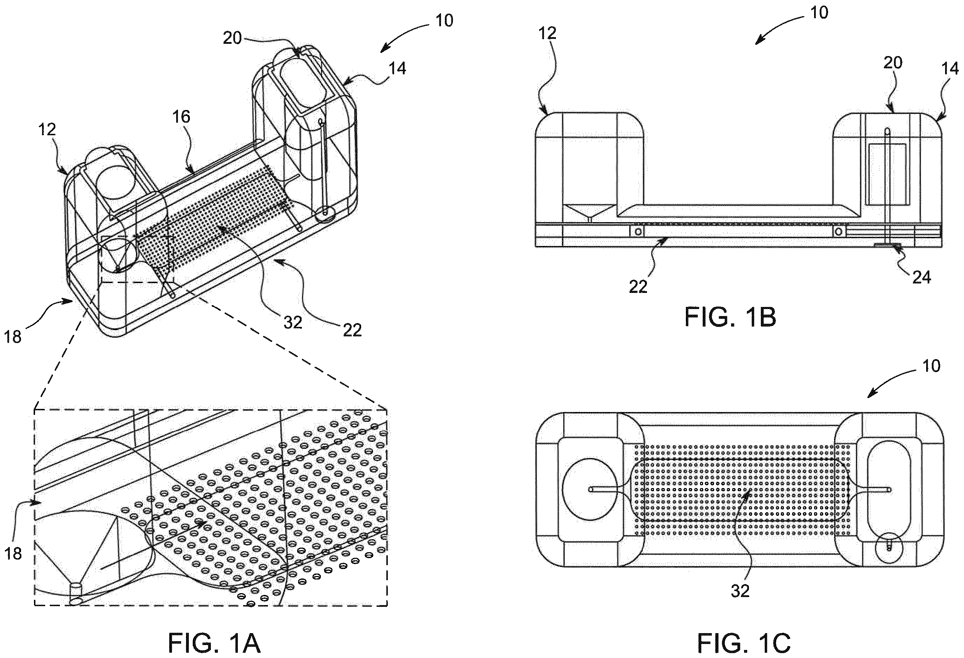

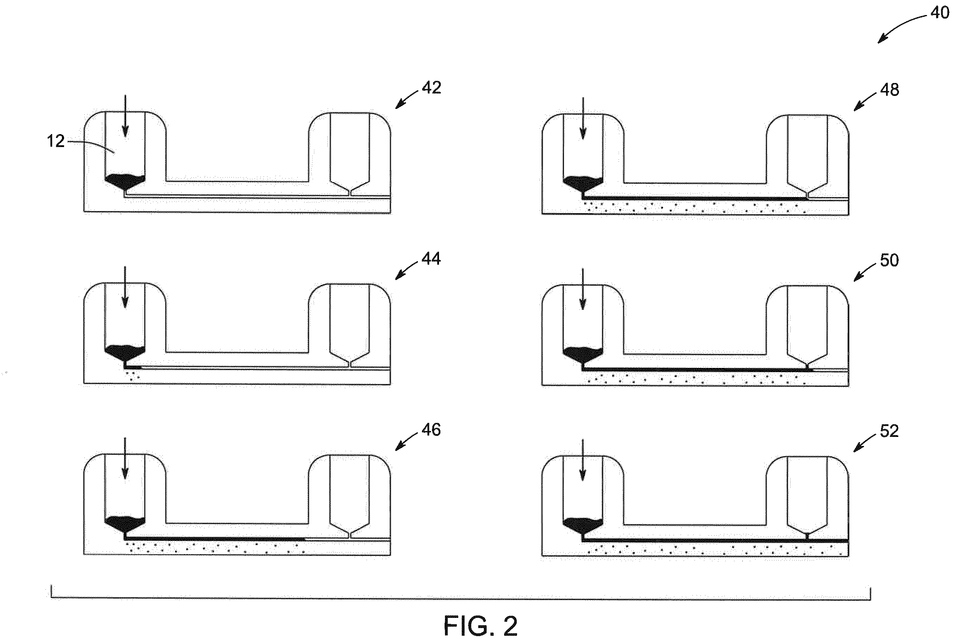

In one or more embodiments, a device is configured to separate particulates present in a fluidic sample. The sample may comprise particulates dispersed in a base fluid and the particulates have a relative density difference compared to the base fluid. In these embodiments, the device for separating particulates from the base fluid comprises a microchannel of length 1 and height h disposed between a fluid inlet and a fluid outlet; a microporous body defining at least a portion of the microchannel; and a collection chamber on an opposing side of the microporous body. The particulates and a portion of the base fluid traverse the microporous body under the influence of an external force field, and are entered and collected in the collection chamber. The microporous body operationally generates a fluid flow regime comprising a first fluid flow having a first flow rate through the microchannel and a second fluid flow having a second flow rate through the collection chamber and the second flow rate is a fraction of the first flow rate. Non-limiting examples of an embodiment of the device are shown in FIGs 1A, 1B and 1C and the related methods steps are shown in FIG. 2.

The term microchannel is used to describe the channel into which the particulates dispersed in a base fluid are introduced. As noted, the microchannel comprises an inlet and an outlet; and one or more walls, wherein the one or more walls comprise a microporous body, such as a microporous surface. The inlet of the microchannel may be configured to receive a sample, such as a biological sample, a water sample or an oil sample. The inlet and the outlet may be present, without limitation at the ends of the channel. The microchannel may comprise one or more walls, and a microporous surface constitutes the one or more walls of the microchannel.

The microchannel is further described as microfluidic channel since at least one of the dimensions of the microchannel is appropriately measured in microns. In various embodiments of the device, a microchannel has a length 1 and a height h. In some embodiments, the microchannel is a horizontal channel configured to permit the flow of a fluid at a predetermined flow rate. The device may be set for a specific flow rate as desired before operating the device. Typically, the microchannel has a length appropriately measured in units larger than microns, for example millimeters (mm), centimeters (cm) or meters (m). In some embodiments of the device, the microchannel has a length 1 between 5 mm and 25 cm. In another embodiment, the microchannel has a length 1 between 10 mm and 10 cm. In yet another embodiment, the microchannel has a length 1 between 10 mm and 25 mm.

The microchannel may be of a regular shape (for example cylindrical) and be of uniform height h. In one embodiment, the average height of the microchannel is between about 1 and about 1000 microns ($\mu$m). In an alternate embodiment, the average height of the microchannel is between about 10 and about 500 microns. In yet another embodiment, the average height of the microchannel is between about 20 and about 250 microns. The microchannel may be of an irregular shape (for example a channel defined in part by an undulating wall) and be characterized by a plurality of heights h. Typically, however, the microchannel is rectangular in shape and is defined on three sides by walls enclosing the microchannel and on a fourth side by the microporous body.

As noted, the microchannel comprises a microporous body, in some embodiments, the microporous body is a microporous surface. In one embodiment, the microporous body constitutes one or more walls defining the microchannel. The microporous body may be a membrane or a solid body through which holes have been created. In one or more embodiments, the microporous body comprises pores originating at a first surface of the microporous body and terminating at a second surface of the microporous body. For example, pores traversing a film may be created by chemical etching techniques and/or laser ablative techniques.

The term "microporous" is used herein because the pores have dimensions appropriately measured in microns. In one embodiment, the pores have an average diameter between about 1 micron and about 500 microns. In an alternate embodiment, the pores have an average diameter between about 10 microns and about 250 microns. In yet another embodiment, the pores have an average diameter between about 20 microns and about 100 microns. In one embodiment, the porosity of the microporous body is between about 10 and about 75 percent. In an alternate embodiment, the porosity of the microporous body is between about 20 and about 65 percent. In yet another embodiment, the porosity of the microporous body is between about 30 and about 60 percent.

As noted, in one embodiment, the microporous body may be a microporous film such as a monofilament screen or mesh made from, for example, polyester, nylon, polypropylene. Alternatively, the microporous body may be a chemically-etched KAPTON, titanium, or NiTinol film. In one embodiment, the microporous body is a laser etched organic film made from an organic polymeric material such as KAPTON.

As noted, the microchannel is disposed between a fluid inlet and a fluid outlet. Fluid enters the microfluidic separation device via the fluid inlet as unprocessed fluid, travels the length of the microchannel to the fluid outlet. During the passage of fluid from the inlet to the outlet, particulates migrate out of the microchannel and enter into the collection chamber under the influence of the fluid flow through the microchannel and one or more additional forces such as the ambient gravitational field, an applied force field, or buoyancy forces. The action within the microchannel converts the unprocessed fluid introduced at the fluid inlet into processed fluid merging at the fluid outlet. In one embodiment, the fluid inlet is configured to receive and hold a fluid sample comprising particulates dispersed within a base fluid, and then to deliver the fluid sample to the microchannel under the influence of a device component, for example a vacuum line applied to the fluid outlet.

As noted, the device comprises a collection chamber in fluid communication with the microchannel configured such that the collection chamber is situated on an opposing side of the microporous body. Typically, the collection chamber is configured such that every pore of the microporous body enables direct fluid communication between the microchannel and the collection chamber. The portion of the microchannel-microporous body interface is referred to herein as the separation zone, wherein the condition of direct fluid communication between the microchannel and the collection chamber through each of the pores is met. The collection chamber is configured to collect particles, which sediment from the fluid sample during processing. For example, the collection chamber collects the cells from a blood sample that is loaded to the microchannel of the device. In the collection chamber, the fluid may be in a quasi-static flow during operation. In some embodiments, the particles sediment and pass through the pores of the microporous surface and are entrapped within the collection chamber. The collection chamber may comprise one or more outlets for collecting the particles which sediment through the microporous surface. The outlet may be connected to a conduit or a syringe to retrieve the particles from the collection chamber of the device. For example, in the case of a blood sample, the separated cells are recovered from the collection chamber using a tube or syringe for downstream applications. In one or more embodiments, the collection chamber is coupled to a pump to recover the particles from the collection chamber during operation.

In various embodiments, the collection chamber is primed, may be partially or completely filled with a priming fluid (e.g. buffer solution) prior to the introduction of the unprocessed fluid into the microchannel. In some embodiments, the collection chamber is filled with a priming fluid prior to initiate a flow through the microchannel. As fluid flows through the device, a first flow regime is established in the microchannel and a second flow regime is established in the collection chamber, the second flow regime being characterized by a lower overall flow rate than that of the first flow regime. In various embodiments, an average time required for the particles to sediment through the microporous body is less than the time required for the particles to transit across the length 1 of the microchannel.

In one or more embodiments, the device further comprises an applied force field across one or more dimensions of the microchannel, for example across the height h of the microchannel to cause the particles to pass through the microporous surface and into the collection chamber. The applied force field may function across the height h of the microchannel under operating conditions of the device. The applied force field may decrease the time required for the particles being impelled through the microchannel by the moving fluid at relatively high flow rate to sediment through the microchannel and be trapped within the collection chamber, wherein the fluid flow rate is relatively low.

In one or more embodiments, an external force causes the particulates to migrate through the microporous body. For example, the external force may be ambient gravity at times herein referred to as ambient gravitational forces. In some embodiments, the applied force field is selected from a magnetic field, an electric field, an electrophoretic field or combinations thereof. In an alternate embodiment, the external force may be a combination of the ambient gravitational forces present together with an applied force field, such as an applied electric field or magnetic field. In an alternate embodiment, the forces causing the particulates to migrate across the microporous body are exerted by the fluid being processed. For example, buoyancy forces may dominate gravitational forces in the separation of oil in water emulsions. In some embodiments, the device further comprises one or more controllers for controlling the applied force field.

In one embodiment, the microfluidic separation device is configured such that the ambient gravitational force acts across the height h of the microchannel and causes particulates dispersed within the base fluid to sediment through the microporous body and into the collection chamber.

The particulates dispersed in the base fluid and traverse together with a portion of the base fluid itself under the influence of passive and/or active forces through the microporous body and are separated from the base fluid. The particulates and a portion of the base fluid are collected in a collection chamber disposed on an opposing side of the microporous body. The fluid sample comprises a plurality of particles in a fluid base, wherein the particles may have a relative density difference compared to the fluid base.

In various embodiments, the separation of particulates from the base fluid occurs as the fluid is flowing through the microchannel for processing. Having traversed the microporous body, the particulates continue to migrate away from the microporous body under the influence of the passive and/or active forces. The base fluid is typically much less susceptible to the influence of the passive and/or active forces as compared to the particulates, and in various embodiments the base fluid entering the collection chamber may remain in relatively close proximity to the microporous body, and is subject to return to the microchannel by re-traversing the microporous surface. This dynamic of particulates and base fluid traversing and base fluid re-traversing the microporous surface creates a flow regime within the collection chamber which has a lower flow rate relative to the flow rate of the fluid being processed through the microchannel. In some embodiments, the microporous surface operationally generates a fluid flow regime comprising a fluid with high flow rate flowing through the microchannel and a fluid with low flow rate flowing through the collection chamber.

As noted, the device for separating particles from a fluid sample comprises a plurality of particulates having a relative density difference when compared to the fluid base. The relative density difference, at least in part, enables the particles to sediment through the microporous surface. The separation of particles using the device and the associated methods, at least in part, is based on sedimentation of the particles through a microporous body (surface).

Sedimentation is one of the simplest methods of cell or particle separation, wherein the separation is based on the density and size of the particle itself. However, sedimentation is typically only thought of as a useful method of high speed cell separation if coupled to a centrifuge and/or density gradient medium (DGM). In the device, as noted, the force which causes the particulates to traverse the microporous body may be an active force such as an applied electric field, a passive force such as the ambient gravitational field, or a combination thereof. For example, in cases where the force of gravity augments the action of an externally applied electric field in inducing particulate movement or sedimentation. In some cases, sedimentation rates are too low to be useful for applications, such as blood cell separation from plasma, or separation of particulate impurities from water. Thus, separation of particulates using the ambient gravitational field or natural particle buoyancy at useful rates has remained an objective.

In the device of FIG. 1A to 1C, the sedimentation rate of the particles in suspension is proportional to the centrifugal force applied to the particles, where

(equation 1):

$$v = \frac{d^2 (\rho_p - \rho_l)}{18\eta} \; x \; g_c$$

v is the sedimentation rate, d is the diameter of the particle, $\rho_p$ is the density of the particle, $\rho_l$ is the density of the DGM, $\eta$ is the viscosity of the DGM, and $g_c$ is the centrifugal force. In the case where the centrifugal force is simply equal to gravity, sedimentation of blood components is slow with erythrocytes settling at ~ 1 micron/s. Previously, this has limited the utility of the earth's gravitational field (g) for high speed, sedimentation-based cell separation, and necessitated the equipment intensive centrifugation approach. As an example, centrifugal forces of 10 - 800 x g are typically applied to blood to separate out specific cell types, which are stratified across the DGM. With the application of the excess centrifugal force, separation can take place on the order of minutes. A simpler and automated separation is enabled by the claimed device that can use only the earth's gravitational field to achieve similar separation rates. It should be noted that other forces would follow a similar relationship where centrifugal, or gravitational force fields could be substituted without limitation for magnetic, electric, or dielectric fields.

As noted, the sample loaded to the device may be processed in the microchannel, as the particles or cells from a fluidic sample or oil from a water sample may be separated and sediment through the microporous surface and enter into the collection chamber. The particles, cells or other materials present in the fluidic sample may be of interest, which are collected or captured in the collection chamber and described herein as "captured particles" or "captured cells". The captured particles or captured cells may be recovered from the collection chamber of the device, which are described at times herein as "recovered particles" or "recovered cells". In some embodiments, the recovery of particles or cells is less than 100%, wherein the number of captured particles or captured cells in the collection chamber is different than the number of recovered particles or recovered cells.

The fluid that flows through the microchannel and emerges at the fluid outlet after removal of at least a portion of the particulates in the unprocessed fluid (at times herein referred to as the "loaded sample") may be recovered from the device's fluid outlet. The fluid recovered from the fluid outlet may be referred to herein as "processed sample" and/or "processed fluid". In some embodiments, the processed sample may be a sample of interest. For example, the processed water sample recovered after removal of the particles is purified water, which is a sample of interest. In some other embodiments, the processed sample may be a waste product. For example, in case of a purification of cells from a whole blood sample, the plasma generated as "processed sample" is collected to a waste chamber and the sample of interest may be the recovered blood cells. In some other examples, the processed plasma may be a sample of interest, depending on the user requirement. In one or more embodiments, the processed sample is collected from the device outlet, wherein the outlet is coupled to a pump to drive out the processed sample.

As noted, the device is configured such that the microporous surface operationally generates a fluid flow regime comprising a high flow rate portion flowing through the microchannel and a low flow rate portion flowing through the collection chamber. The microporous surface effectively generates a resultant force derived from a fluid drag force in the direction of fluid flow across the length 1 of the microchannel and a force for particle sedimentation across the height h of the microchannel. In some embodiments, the resultant force favors sedimentation and captures the particles in the collection chamber. The fluid flow rate above the microporous surface may be high, which allows processing of larger sample volume using the device compared to the sample volume typically used for known devices.

The device may be configured, such that the time required for the particles to sediment through the microporous surface is less than the time required for the particles to transit across the length 1 of the microchannel. The particle capture efficiency and/or volumetric throughput may be improved by either increasing the length 1 or decreasing the height h of the microchannel. An increase in length 1 of the microchannel increases the time available for the particles to interact with the microporous surface. As noted, "capture efficiency" refers to a probability function, dependent on the average number of particles interacting with the pores during transit through the device and the probability of particle passage through a pore for each such interaction. The probability of particle passage through the pore for each interaction may depend on the ratio of particle/pore diameters.

In addition to particle collection, the device may be configured to distinguish particles with respect to size, sedimentation velocity, or density. The particles with different size may be separated using the device. The particles having different sedimentation velocity may also be separated using the device. In some embodiments, the particle has an average diameter between 1 and 250 $\mu$m. As the microporous surface comprises pores with an average diameter in a range of 1 to 500 $\mu$m, the particles that are smaller than the pore size pass through the microporous surface. The particles, which are passed through the pores of the microporous surface, are captured in the collection chamber.

One embodiment of a device for separating one or more cells of average diameter (d) from a blood sample, comprises a microchannel of length 1 and height h comprising an inlet and an outlet; a microporous surface with an average pore diameter (p) and porosity (q), on one or more walls of the microchannel; a collection chamber on the opposing side of the microporous surface; and an applied gravitational field across the height h of the microchannel to sediment the cells through the microporous surface into the collection chamber. In this embodiment, as noted before, the microporous surface operationally generates a fluid flow regime comprising a high flow rate portion that flows through the microchannel and a low flow rate portion that flows through the collection chamber. In this embodiment, an average time required for the cells to sediment through the microporous surface is less than the average time required for the cells to transit across the length 1 of the microchannel.

As illustrated in FIG. 1 A, the device (front view) 10 comprises a fluid inlet 12, a fluid outlet 14 and a microchannel 16. In some embodiments, the term "microchannel" is used interchangeably with the term "separation channel". The fluid inlet 12 may at times function as and be referred to as an inlet well which connects to the microchannel. In the embodiment shown, fluid inlet 12 is configured as an inlet well in fluid communication with microchannel 16 via conduit 18. The microchannel 16 is bounded on its lower side by microporous body 32. The device 10 also comprises a collection chamber 22 on an opposing side of the microporous body 32.

As shown in FIG. 1 B, in one exemplary embodiment, a side view of the device 10 comprises a collection chamber 22. In some embodiments, a vacuum or a syringe 24 is coupled to the collection chamber of the device to pull a sample loaded to the device. In another embodiment, the device 10 comprises a vacuum 20 coupled to the device outlet 14 to pull the sample loaded to the device to a waste-tub or waste-chamber and drive the sample fluid through the device 10. In other embodiments, the fluid-flow across the device is accomplished using a positive pressure applied to the device inlet 12 for sample load. In some embodiments, a gravity-driven flow provided through the device-inlet.

In some embodiments, a top view of the device 10 comprises a microporous surface 32, as shown in FIG. 1 C. The pore size of the microporous surface is small enough to provide distinct flow regimes above and below the microporous surface, whereas the pore size is large enough to allow sedimentation of the particles through the pores and capture within the collection chamber.

The device enables high speed separation without using a centrifuge or additional equipment. In an exemplary embodiment of the device 10, a high velocity flow-stream flows across a wide sedimentation area of the microchannel 16 over the microporous surface 32. For example, the microchannel comprises a sedimentation area of 10 mm x 400 mm over the microporous surface. In one embodiment, the flow-stream within the microchannel 16 extends across the microporous surface 32, which covers the collection chamber 22. Unlike a filtration device, a pressure drop across the microporous surface 32 is significantly minimized as the fluid stream enters into the microchannel 16 through the opening 18 and spreads over the wide area of the microporous surface 32. The majority of the fluid-flow occurs over the microporous surface 32. The device is configured such that the fluid sample is entered into the microchannel 16, spread over the microporous surface 32. The particles or cells of the fluid sample are sediment through the pores of the microporous surface, and are trapped into the collection chamber 22 underneath the microporous surface 32.

In an example of a method for separating one or more particles from a fluidic sample, the method comprises loading the sample to a device, wherein the device comprises a microchannel of length 1 and height h, comprising an inlet and an outlet; a microporous surface constitutes one or more walls of the microchannel; a collection chamber on an opposing side of the microporous surface, and an applied force field across the height h of the microchannel to sediment the particles through the microporous surface and capture into the collection chamber. The method further comprises contacting the sample with the microporous surface; generating a fluid flow regime comprising a high velocity portion flowing through the microchannel and a low velocity portion flowing within the collection chamber; sedimenting the particles through the microporous surface into the collection chamber; collecting the particles in the collection chamber under the applied force field and retaining a fluid in the microchannel; and driving out the retained fluid through the outlet of the microchannel. An average time required for the particles to sediment through the microporous surface is less than the average time required for the particles to transit across the length 1 of the microchannel. The fluid recovered after sedimentation of the particles or cells from the sample comprises a reduced number of particles or cells compared to the number of particles or cells initially present during loading of the sample.

In one embodiment, the sample is a whole blood sample, wherein the method is employed to separate one or more cell types from the blood sample. In this embodiment, the method comprises loading the blood sample to the device comprising a microchannel with a microporous surface and a collection chamber, contacting the blood sample to the microporous surface; generating a fluid flow regime comprising a high velocity portion flowing through the microchannel and a low velocity portion flowing through the collection chamber; sedimenting the cells through the microporous surface and capturing into the collection chamber under the applied force field and retaining a plasma fluid in the microchannel. The retained fluid, here plasma, is driven out through the outlet of the microchannel, wherein a time required for the cells to sediment through the microporous surface is less than the time required for the cells to transit across the length 1 of the microchannel.

Examples of methods for separation of cells from a whole blood sample are illustrated in FIG. 2. FIG. 2 illustrates separation of cells and plasma from a 0.5mL sample of whole blood. The method resolves the challenge of using gravity to separate cells in a high throughput manner. As shown in FIG. 2, the method 40 encompasses various steps of an exemplary embodiment. In step one 42, 0.5 mL sample of whole blood is loaded to the device 10 through the inlet 12. In step two, 44, a vacuum is applied to the outlet 14 of the device to drive the sample through the device. The sample enters to the device from the inlet 12, passing through the microchannel 16 and exits from the device through the outlet 14. In step three, 46, the device runs until the collection chamber is filled with the captured cells from the whole blood sample. Step four, 48, includes the sedimentation of the cells to the collection chamber and driving out the plasma from the device outlet 14 to a waste chamber or to a second collection chamber. In step five, 50, the cells that entered to the collection chamber 22 due to sedimentation through the pores, are visible in the collection chamber from the bottom. In step six, 52, the collection chamber 22 is opened through an outlet to drain the collected cells out form the device 10.

The spreading of the fluid flow stream across the wide separation surface provides a significant opportunity for interaction of the particles with the surface. The addition of a static collection chamber 22 allowed sedimentation of the particles into the collection chamber, without traversing the particles across entire length 1 of the microchannel, unlike standard centrifuge tubes or large sedimentation tanks. The pressure drop across the microporous surface is minimized enough such that re-entry of the particles into the high speed flow-stream and loss of particles into the waste chamber is minimized, which is demonstrated by showing clear plasma in FIG. 2, step four, 48. The efficiency of the separation and the relative performance of the device compared to the filtration technology are significantly high.

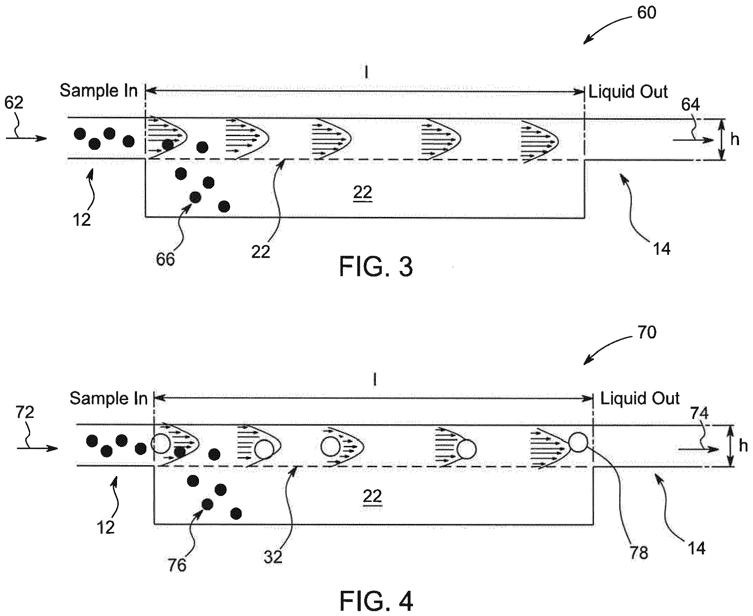

FIG. 3 illustrates one embodiment of the device 60 under operating condition, wherein the unprocessed fluid (sample) 62 containing particles 66 enters into the device through the device inlet 12 and the fluid flow 64 exits from the device through the device outlet 14. The particles 66 are sediment through the porous surface 32 of the microchannel and trapped into the collection chamber 22. In this embodiment, the percentage of particles 66 is significantly reduced in the processed fluid 64, as the particles 66 are sediment through the microporous surface and trapped within the collection chamber 22. The length and height of the microchannel is 1 and h, respectively.

FIG. 4 illustrates an additional embodiment of the device 70 under operating condition, wherein the unprocessed fluid (sample) 72 comprising smaller particles 76 and larger particles 78. The sample 72 enters into the device through the inlet 12 and the processed fluid 74 exits from the device through the outlet 14. The larger particles 78 are larger than the diameter of the pores within the microporous surface 32. The particles 78 cannot pass through the pores to trap into the collection chamber 22, hence particles 78 are retained within the microchannel in the processed fluid 74. In this embodiment, the smaller particles 76 sediment through the microporous surface 32, collect to the collection chamber 22, and are separated from the larger particles 78. The length and height of the microchannel is 1 and h, respectively. In this device, there is a limited fluid flow across the microporous surface 32 and particles enter into the collection chamber 22 through sedimentation, unlike a tangential flow filtration process.

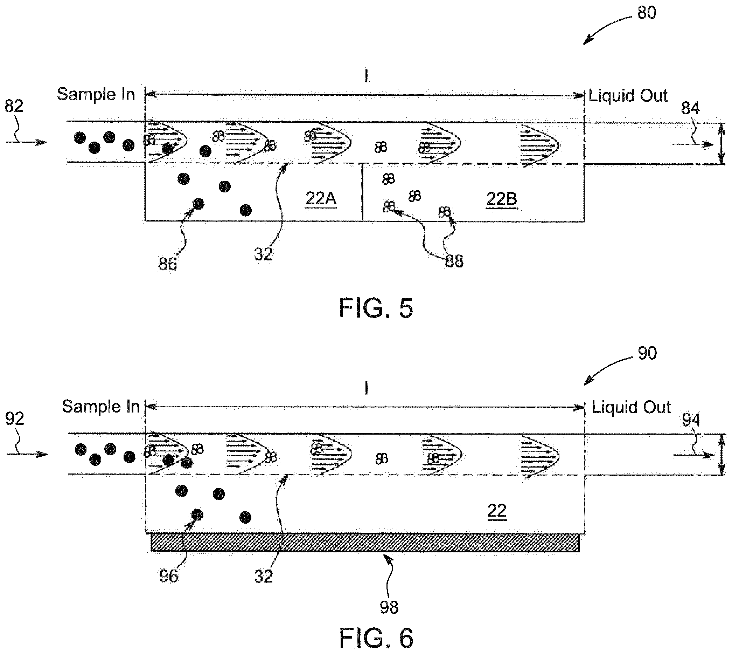

FIG. 5 shows another embodiment of the device 80 under operating condition. In this embodiment, the unprocessed fluid (loaded sample) 82 comprises a plurality of particles 86 and 88, wherein the particles 86 and 88 have different sedimentation rates. The particles 86 and 88 are captured in segmented portions of the collection chamber 22 A and 22 B respectively. In this embodiment, the similar or same size particles may be separately sediment in two different segments of the collection chambers 22 A and 22 B based on the particle's sedimentation rate. The particles 86 having higher sedimentation rate sediment faster and collect to the segment 22 A of the collection chamber closer to the inlet. The particles 88 having lower sedimentation rate sediment later and collect into the segment 22 B of the collection chamber closer to the outlet. The processed sample 84 is driven out from the device outlet. The length and height of the microchannel is 1 and h, respectively.

Another embodiment of the device 90 is shown in FIG. 6, wherein a fluid sample 92 is loaded to the device and a processed fluid 94 is recovered from the device outlet. In this embodiment, an additional force, such as a magnetic force field 98 is applied to sediment the particles 96 through the microporous surface 32 and are trapped into the collection chamber 22. The magnetic field is applied across the microporous surface to allow only certain particle types to enter into the collection chamber 22. For example, the particles 96 pass through the micropores have a magnetic property. A population of cells having magnetic property may also be separated from a fluid base using this embodiment of the device.

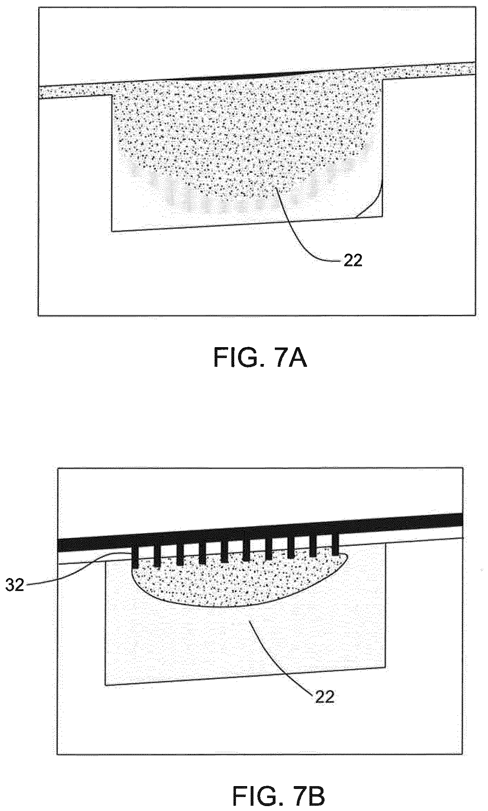

FIG. 7 A is an image from a computational fluid dynamic (CFD) model showing fluid flow pattern through a representative device without a microporous surface. The representative device is a channel with one inlet and an outlet, without any microporous surface in it. The representative device without a microporous surface has the same dimension of inlet, outlet, channel length, channel height as of the present device. A significant portion of the fluid flows through the device enters into the collection chamber through the microchannel. FIG. 7 B is computational fluid dynamic (CFD) model showing fluid flow pattern through the device with a microporous surface 32, wherein distinct flow regimes are generated above the microporous surface 32 versus below the surface 32. The presence of microporous surface provides a fluid resistance, which may limit the fluid to flow into the collection chamber.

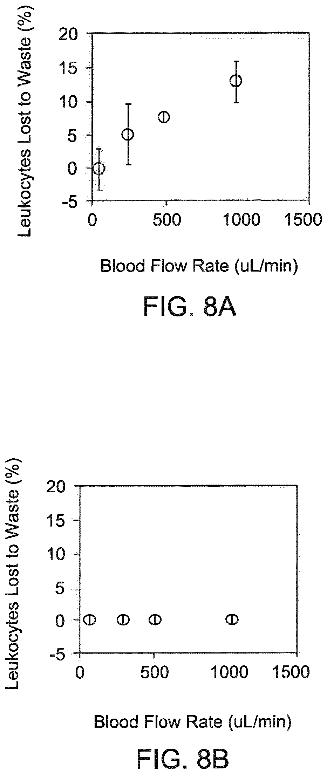

FIG. 8A is a graph showing loss of white blood cells (or leukocytes) to the fluid that recovered from the device outlet with increasing fluid flow rate using a representative device without a microporous surface, as described above. A significant cell loss occurs when the microporous surface is absent. FIG. 8B is a graph showing minimum loss of leukocytes to the fluid that recovered from the device outlet with increasing fluid flow rate using a device with a microporous surface, wherein nearly 100% of the leukocytes of the blood are captured in the collection chamber.

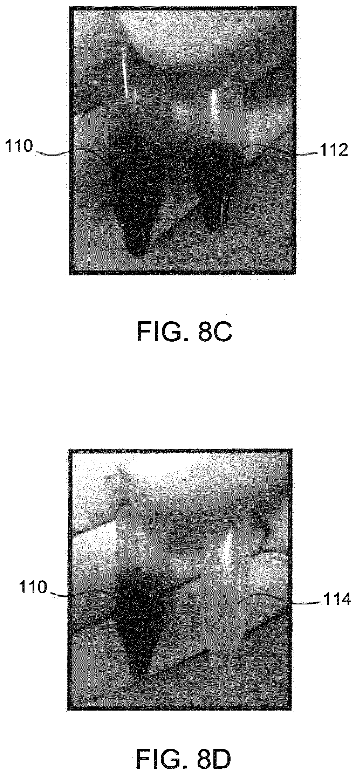

FIG. 8C is an image of a blood sample 110 loaded through a device inlet and a fluid sample 112 recovered from the device outlet using a representative device without a microporous surface, as described above. The image (8C) shows the sample 112 is plasma contaminated with red blood cells. FIG. 8D is an image of a blood sample 110 loaded through a device inlet at a flow rate of 1000 $\mu$l/min and a fluid sample 114 recovered from the device outlet using a device with a microporous surface. The image of 8D clearly shows the recovered fluid 114 is clear plasma, collected from the device comprising a microporous surface.

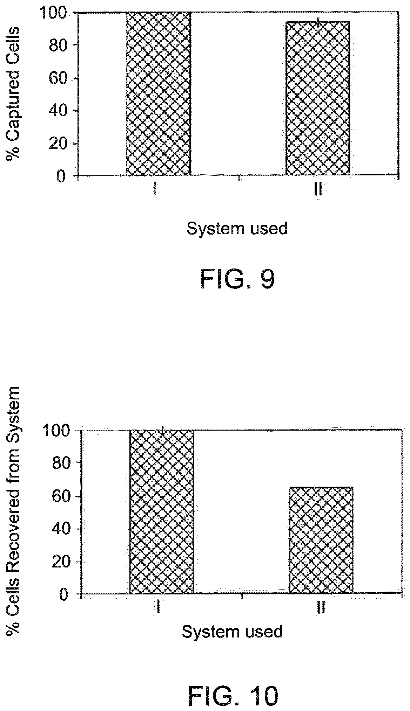

FIG. 9 shows higher cell separation efficiency of the microfluidic separation device (I) compared to the cell separation efficiency using a commercial filtration device designed specifically for capturing white blood cells (II). The cell separation efficiency is measured in terms of capture of the white blood cells in the collection chamber. In addition, a common problem with currently available white blood cell filters is loss of cells due to retention within the filter membrane. In contrast, in embodiment of the present device, the loss of cells is addressed by collection of cells within the liquid filled collection chamber below the microporous surface. FIG. 9 shows the ability of the device that competes with traditional filtration techniques for capturing cells. FIG. 10 shows much higher (-80%) actual white blood cells recovery from the present microfluidic device comprising a microporous surface (I) compared to a commercially available cell filter membrane (II) (-60%). The difficulty in recovering captured cells from the surface of filtration membranes is addressed.

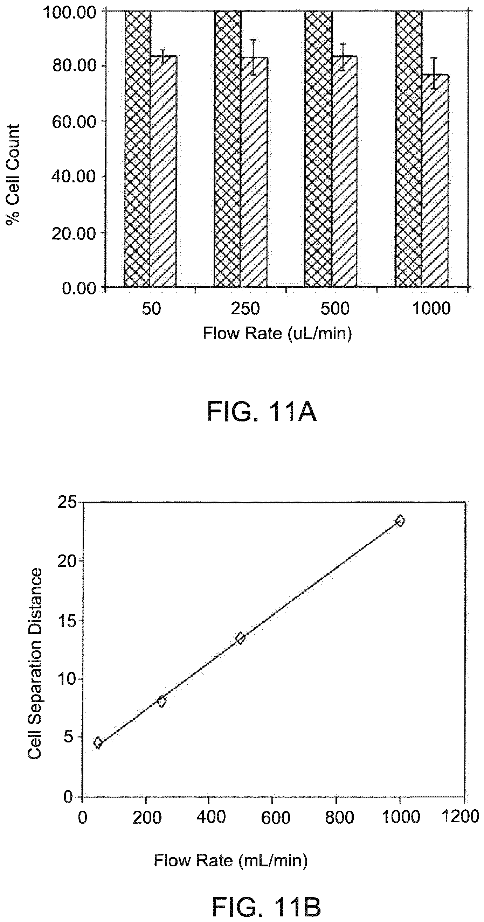

FIGs. 11 A and 11 B are graphs showing the device is operational with higher flow rates compared to a flow rate used by commercially available microfluidic separation devices. The recovery of white blood cells from the processed fluid was consistent for the flow rate range of 50 $\mu$l/min to 1 mL/min. The recovery of cells was not decreased even at 1 mL/min flow rate (FIG. 11A). The percent of cells captured in the collection chamber slightly decreases with increasing flow rate. In the present embodiment of the device, the microporous surface is positioned perpendicular to the fluid flow path. The cells sediment and separate from the fluid flow stream after a certain length (1) of the microchannel. With increase in flow rate, the length of the microchannel that is necessary to achieve separation of the cells is also increased, as shown in FIG. 11 B. The separation distance is estimated by visual observation of red cell front within the device during operation.

As noted, the sample loaded to the device is a fluidic sample. In some embodiments, the fluid may include a biological sample, water sample, aqueous slurry, oil slurry, oil-water emulsion or combinations thereof. As noted, the biological materials used in the embodiments may comprise a physiological body fluid, a pathological body fluid, a cell extract, a tissue sample, a cell suspension, a forensic sample and combinations thereof. In some embodiments, the biological material is a physiological body fluid or a pathological body fluid, such as the fluid generated from secretions, excretions, exudates, and transudates, or cell suspensions such as, blood, lymph, synovial fluid, semen, saliva containing buccal swab or sputum, skin scrapings or hair root cells, cell extracts or cell suspensions of humans or animals. In some embodiments, the physiological/pathological liquids or cell suspensions may be extracted from plants. In one or more embodiments, the extracts or suspensions of parasites, bacteria, fungi, plasmids, or viruses, human or animal body tissues such as bone, liver or kidney. In some embodiments, the sample fluid is a biological sample selected from whole blood, cell extract, tissue extract or combinations thereof. In one embodiment, the sample fluid comprises whole blood.

In one or more embodiments, the particle comprises red blood cells, white blood cells, platelets, biological cells, tissue fragments, metals, minerals, polymers or combinations thereof.

In some embodiments, the device 10 (FIGs. 1A, B, or C) may be a portable or field-able device, so that the biological materials can be collected at any location and loaded into the device for cell separation. In some examples, the device may run using a pump. In one embodiment, the device is packaged with a power source, wherein the entire assembly may be self-contained. In such embodiments, the device is portable, simple, and user friendly compared to existing devices in the market.

The applications for the device 10 (FIGs. 1A, B, or C) include, but are not limited to, therapeutic application, biochemical analysis, proteomics, healthcare related applications, pharmaceutical or biotech research applications, environmental monitoring, in vitro diagnostic and point-of-care applications, or medical devices.

In one or more embodiments, the device 10 (FIGs. 1A, B, or C) is fully automated or partially automated. The automation of the device is required to reduce human intervention during collection of cells. The use of an automated device further helps in minimizing contamination during purification of biological samples, aqueous samples, and oil samples. Fully automatic devices are desirable for various applications, wherein the objective is to purify blood cells, blood serum or water or oil from a sample. An externally located controller may be operationally coupled to the device to drive the device, excluding any manual intervention after application of the biological sample, water or oil to the device-inlet.

In some embodiments, the device is configured to integrate with another device or system, more specifically with an analytical device. As noted, the device may have one or more coupling means through which the device may integrate with another device depending on the requirement. The coupling means may include but is not limited to, an adapter, or a connector. In some embodiments, the device itself is configured to have one or more holders, connecting ports or combination thereof, which mechanically couples the device to another device. The device may be electronically or mechanically coupled to another device for downstream applications.

In one or more embodiments, the device further comprises one or more containers for collecting waste or fluid after separation of the particles or cells. In embodiments, where blood is a sample, the plasma generated after separation of the cells may be collected to a waste chamber. In some other embodiments, when the sample is water or oil, after separation of the particles, the purified fluid is collected to a collection chamber coupled to the outlet. This collection chamber is different than the collection chamber for particles present opposing side of the microporous surface. In one or more embodiments, the non-limiting examples of containers are bag, chamber and vessels. The containers may be disposable or reusable. Various components of the device may be operationally connected to each other using conduits, holder, adapter, or valves. The device may further comprise one or more sensors, such as temperature sensor, pressure sensor, flow sensor or pH sensor, depending on the requirement.

EXPERIMENTAL PART

DEVICE FABRICATION

A microfluidic separation device housing was created using a commercially available rapid prototyping instrument and an ABS-like photopolymer (DSM Somos WaterShed XC 11122). The microfluidic separation device was assembled from three parts, created on the rapid prototyping instrument together with a porous KAPTON film which served as the microporous body or microporous surface, and a set of pressure sensitive adhesive films which joined the parts together and served to create the microchannel. Useful reference may be made to FIG.s 12-14 to better understand the fabrication of the microfluidic separation device.

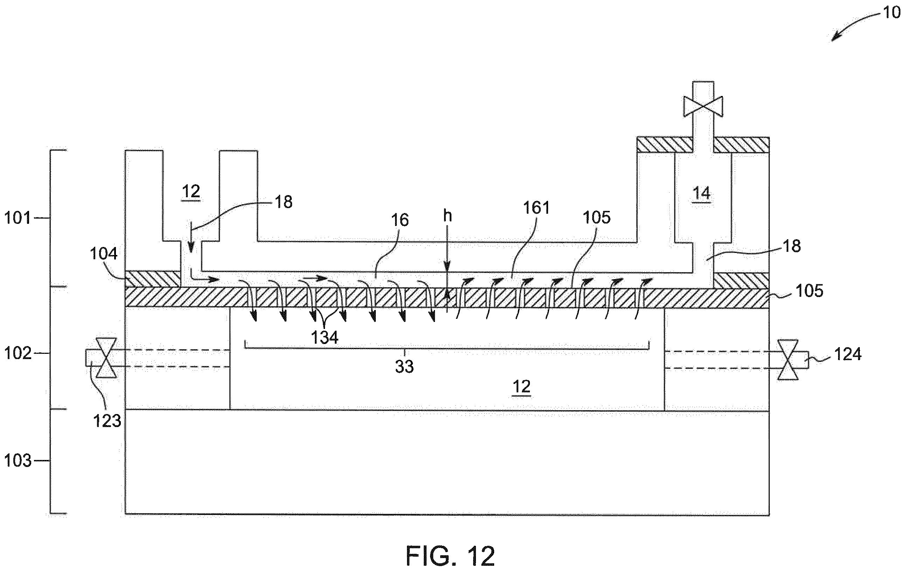

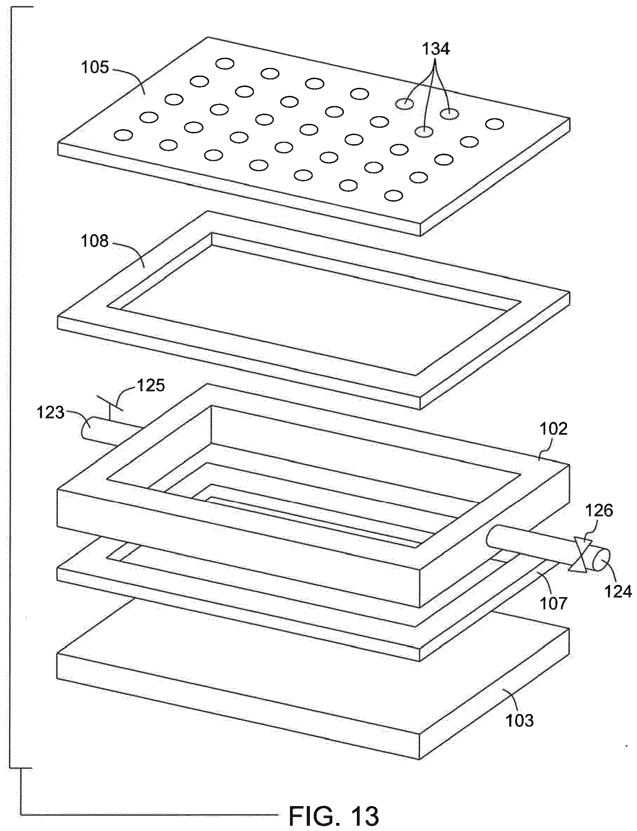

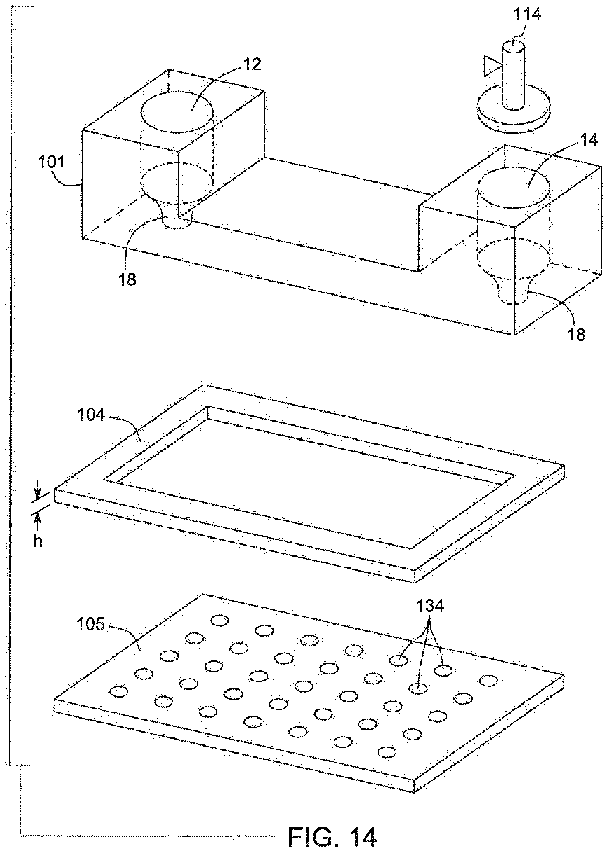

The first part 101 (FIG. 14) comprised a fluid inlet 12 and fluid outlet 14 with slots 18 linking each to microchannel 16 (FIG. 12). The second part 102 (FIG. 14) defined the collection chamber 22 (FIG. 12). A third part 103 (FIG. 13) formed a wall of the collection chamber. A 50 micron ($\mu$m) thick pressure sensitive adhesive 104 (FIG. 14) was used to define the microchannel having dimensions 50 millimeters by 10 millimeters by 50 microns and comprised features cut out using a cutter/plotter (Graphtec Craft Robo ProS). The adhesive film 104 also served to fix the microporous body 105 (FIG.s 12-14) (the porous KAPTON film) to the first part 101. Additional cut adhesive films 106 and 107 were used to fix the second part 102 to the microporous body 105 and the third part 103 respectively. In the embodiment shown microporous body 105 comprises pores 134.

Two different types of microporous bodies were used in the devices. As mentioned, the first type of microporous body was formed from a KAPTON sheet with laser-machined pore arrays having average pore diameter of about 21.7 microns with a 50 micron center-to-center pore spacing. The second type of microporous body employed was a medical grade polyamide woven mesh having 40 micron pores and 40% porosity (SEFAR MEDIFAB, 07-40/40).

The collection chamber 22 defined by second part 102 had dimensions of 40 millimeters by 10 millimeters by 2 millimeters, resulting in a 750 microliter ($\mu$L) holding volume. As configured, the microfluidic separation device could process a total volume of about 0.5 milliliters of blood before the collection chamber reached its maximum cell holding capacity.

DEVICE OPERATION

The microfluidic separation device was equipped with two ports 123 and 124 (FIG.s 13-14) which enabled the device to be primed easily before use. Typically, the device was primed by introducing deionized water through one of the two ports 123 and 124 and completely filing both the collection chamber 22 and the microchannel 16 before use. Alternatively, the device could be primed by flowing deionized water from the fluid inlet and into the microchannel and collection chamber. Typically, the priming liquid could be introduced into the microfluidic separation device without introducing air bubbles.

Once primed, a sample fluid comprising particulates dispersed with a base fluid (such as whole blood comprising blood cells are the particulates) dispersed within blood plasma (the base fluid) was introduced into fluid inlet 12 and was made to flow through the microchannel 16 contact the upper surface of the microporous body by the application of a vacuum on the fluid outlet 14 side of the device. Owing to the gravitational forces present, particulates within the sample fluid flowing within the microchannel tended to sediment downwardly through the pores of the microporous body and into the underlying water-filled collection chamber, wherein the downward motion of the particulates continued. Operation of the microfluidic separation device typically effected at least a substantial separation of particulates and base fluid. The processed fluid, a mixture of the base fluid and water exchanged with the collection chamber was collected in the fluid outlet.

EXAMPLES

Cell Separation-Whole blood or cell-suspensions were used to test collection efficiencies of mammalian cells using the microfluidic separation device. Typical white blood cell dimensions are 10 to 12 microns, and thus the microporous body having 21.7 micron pore diameters provided an about a 2 to 1 ratio of pore diameter to cell dimension. A syringe pump was attached to the fluid outlet used to create a flow of the cell-containing sample in the fluid inlet through the microchannel and into the fluid outlet at defined rates (PicoPlus syringe pump, Harvard Apparatus) from 50 to 1000 microliters per minute ($\mu$L/min). Phosphate buffered saline (PBS), deionized water or cell culture medium were used as priming fluids. Separated base fluid was collected by pipetting from the fluid outlet. Separated cells were recovered from the collection chamber using a syringe. In all cell separation examples disclosed herein, the external force field which caused the cells to traverse the microporous body from the microchannel to the collection chamber was gravity and the microfluidic separation device was oriented such that the passage of cells from the microchannel to the collection chamber was in a downward direction.

Cell collection efficiency was assessed on a SysMex XE2100 Hematology Analyzer and provided red and white blood cell counts from whole blood samples. White blood cell viability and collection efficiency were assessed on a NucleoCounter® *(chemometec) Live/Dead Analyzer. Collection efficiencies were recorded as the ratio of the number of cells introduced through the device fluid inlet 12 (FIG. 12) to the number of cells collected in the collection chamber 22 (FIG. 12). The number of cells actually recovered from the collection chamber was also recorded as there was some additional loss of cells during the transfer of the contents of the collection chamber. Total cell loss was significantly less than losses occurring in standard filtration protocols used for white blood cell capture. Viability of the cells was not affected by passage through the microfluidic separation device over the range of flow rates tested. Cell collection efficiency was assessed at sample flow rates of from 50 microliters per minute to 1000 microliters per minute ($\mu$L/min). In addition, a total surface area of the microporous body necessary to enable efficient cell separation was estimated for each flow rate.

EXAMPLE 1: Analysis of cell-separation using Computational Fluid Dynamics (CFD)

Computational Fluid Dynamics: A finite element method analysis solution to the full Navier-Stokes equation was also performed using Comsol® multiphysics. Velocity fields were extracted for scaled devices (large enough to model ten pores across the microporous surface) with and without the microporous surface. This allowed visualization of the effect of the microporous surface on flow conditions within the collection chamber. The output boundary condition was set to pressure at 0 Pa, inflow velocities were set to match the 50 uL/min experimental conditions. Collection efficiencies over a range of particle/fluid density ratios were estimated using the particle tracing function in Comsol®, and counting the number of particles that entered the pore array. Additional simulations were then run to investigate the effect of changing the particle/pore size ratio for densities that match those reported for white blood cells and plasma in the literature. FIGs. 7A and 7B show CFD model analysis of blood sample passing through a representative device without a microporous surface and a device with a microporous surface, respectively.

Whole blood (0.5 mL) was introduced into to the fluid inlet of a primed microfluidic separation device and caused to flow through the device at a flow rate of 1000 $\mu$l/min. A microfluidic separation device without a microporous body that separate the collection chamber from a microchannel was used as a control. This device had an idealized configuration and fluid dynamics is shown in FIG. 7A in which the microchannel 16 is absent in the separation zone 32. FIG. 7B provides a useful point of reference and shows fluid dynamics using an idealized microfluidic separation device with the microporous body in place and the microchannel extending fully across the separation zone 33, which includes the portion of the microchannel in contact with the microporous body. Fluid flowing into the device shown in FIG. 7A enters the collection chamber without being constrained by the presence of a microporous body. As a result a complex flow regime is created in the collection chamber. In the absence of the microporous body, cells are both captured within and pass through the collection chamber. In the experiments carried out in the absence of the microporous body the efficiency of cell separation was strongly dependent of sample flow rate through the device.

EXAMPLE 2: Recovery of white blood cells from whole blood using both of the device and a representative device without a microporous body

Whole blood (0.5 mL) was introduced into to the fluid inlet of a primed microfluidic separation device and caused to flow through the device at a flow rate of 50, 250, 500 or 1000 $\mu$l/min. The microfluidic separation device identical to that used in present Example with the exception that no microporous body to separate the collection chamber from a microchannel was used as a control. Data are gathered in FIG. 8A for cell separation using a device without a microporous body, which shows a steady decline in cell separation efficiency as the loss of cells increases to the waste with increasing flow rate. In contrast, FIG. 8B shows high cell separation efficiency and minimum loss of cells to the waste with increasing flow rate while using a device with a microporous body. The loss of white blood cells (or leukocytes) to the fluid that recovered from the device outlet was increased with increasing fluid flow rate (FIG. 8A). While only a minimal loss of leukocytes to the fluid that recovered from the device outlet was observed, even with increasing fluid flow rates (FIG. 8B). A significant number of leukocytes of the blood was captured and recovered in the collection chamber of the device.

EXAMPLE 3: Recovery of red blood cells from whole blood using the microfluidic device and a representative device without a microporous body

Red blood cell separation was carried out with a flow rate of 1000 $\mu$l/min on a sample consisting of 0.5 mL of whole blood using a primed microfluidic separation device disclosed herein. Separation of red blood cells from the processed fluid collected in the fluid outlet was essentially quantitative. The relative performance of the device was compared with a representative device without a microporous body. Whole blood (0.5 mL) was introduced into to the fluid inlet of a primed microfluidic separation device. The microfluidic separation device identical to that used in present Example with the exception that no microporous body to separate the collection chamber from a microchannel was used as a control. The plasma recovered from the outlet of the device is transparent and clear fluid without contamination of red blood cells, as shown in FIG. 8D, compared to the plasma collected from the outlet of the representative device, which is turbid and contaminated with red blood cells, as shown in FIG. 8C.

The role of the microporous body is evident in FIG. 8C (microporous body absent) and FIG. 8D (microporous body present) wherein in each case element 110 is the starting whole blood sample and elements 112 and 114 are the processed fluid collected in the fluid outlet in the presence and absence of the microporous body respectively. FIG. 8C shows clearly the presence of red blood cells which escaped capture in the separation zone in the processed fluid collected in the fluid outlet. FIG. 8D show the processed fluid 114 as essentially free of red blood cells.

EXAMPLE 4: Separation of blood cells from a whole blood

A whole blood sample (0.5 mL) was introduced into the primed microfluidic separation device configured as in FIG. 12 and was made to flow through the microchannel at a flow rate of 250 $\mu$l/min. The processed fluid was analyzed and shown to be free of white blood cells (FIG. 9, left column). The results obtained were compared to the performance of a commercial filter designed for capturing white blood cells. FIG. 9 illustrates the effectiveness of the present invention in overcoming a common problem associated with filtration techniques wherein cell separation efficiency is limited by a tendency of the filter to bind cells.

FIG. 10 illustrates that the actual recovery of blood cells from the collection chamber and microporous body of the microfluidic separation device provided by the present invention is enhanced relative to the commercial filter. The recovery of cells from the blood sample was about 80% using the microfluidic separation device of the present invention, whereas cell recovery using the benchmark filter was only about 60%.

Additional experiments were carried out at higher and lower flow rates using the microfluidic separation device configured as in FIG. 12. Results are gathered in FIG. 11A, which show that the processed fluid collected from the fluid outlet is substantially free of blood cells and that a significant percentage of the blood cells are recoverable from the device following processing. Thus, even at a flow rate of 1000 μl/min (FIG. 11 A) the efficiency at which white blood cells were removed from the blood plasma was not decreased relative to the results obtained at a 50 μl/min flow rate. A commercial flow filter was unable to fully separate out cells from 0.5 mL of blood, as the filter stalled at 5 PSI running pressure due to occlusion of the filter pores with captured cells (data not shown). The commercial benchmark also required a stack of 5 filters having a 15 mm diameter each (883.5 mm² area) in order to achieve separation efficiencies comparable to those observed for the microfluidic separation device provided by the present invention.

**Claims**

1. A method for separating particulates dispersed within a base fluid and having a relative density difference compared to the base fluid, comprising:

    providing a separation device (10) comprising:
    a microchannel (16) of length 1 and height h disposed between a fluid inlet (12) and a fluid outlet (14); a microporous body (32) defining at least a portion of the microchannel; and a collection chamber (22) on an opposing side of the microporous body; wherein the particulates and a portion of the base fluid traverse the microporous body under the influence of an ambient gravitational force, and are entered and collected in the collection chamber;
    introducing a sample of unprocessed fluid comprising particulates dispersed within a base fluid into the microchannel (16) via the fluid inlet (12);
    separating at least a portion of the particulates from the unprocessed fluid to provide a stream of processed fluid at the fluid outlet (14); and
    recovering at least a portion of the particulates initially present in the unprocessed fluid in the collection chamber (22); **characterized in that** the particulates and a portion of the base fluid traverse the microporous body (32) under the influence of an ambient gravitational force with the exclusion of other external force, and are entered and collected in the collection chamber (22); and

    wherein the microporous body operationally generates a fluid flow regime comprising a first fluid flow having a first flow rate through the microchannel (16) and a second fluid flow having a second flow rate through the collection chamber (22) and the second flow rate is a fraction of the first flow rate, and further comprising a step of priming the device prior to introducing the unprocessed fluid into the microchannel (16).

2. The method of claim 1, further comprising re-traversing the fluid through the microporous body (32) and re-entering the microchannel (16).

3. The method of claim 1, wherein the unprocessed fluid comprises one or more of whole blood, a cell extract, or a tissue extract.

4. The method of claim 1, wherein the particulates are blood cells.

5. The method of claim 1, wherein the processed fluid comprises blood plasma.

**Patentansprüche**

1. Verfahren zum Abtrennen von Teilchen, die in einem Basisfluid dispergiert sind und im Vergleich zum Basisfluid einen relativen Dichteunterschied aufweisen, umfassend:

    Bereitstellen einer Trennvorrichtung (10), welche umfasst:
    einen Mikrokanal (16) der Länge I und Höhe h, der zwischen einem Fluideinlass (12) und einem Fluidauslass (14) angeordnet ist; einen mikroporösen Körper (32), der mindestens einen Teil des Mikrokanals definiert; und eine Sammelkammer (22) auf einer dem mikroporösen Körper gegenüberliegenden Seite; wobei die Teilchen und ein Teil des Basisfluids den mikroporösen Körper unter dem Einfluss einer umgebenden Schwerkraft durchdringen und in die Sammelkammer geführt und in dieser gesammelt werden;

Einleiten einer Probe unbehandelten Fluids, das in einem Basisfluid dispergierte Teilchen umfasst, über den Fluideinlass (12) in den Mikrokanal (16);

Abtrennen mindestens eines Teils der Teilchen vom unbehandelten Fluid, sodass am Fluidauslass (14) ein Strom behandelten Fluids bereitgestellt wird; und

Gewinnen mindestens eines Teils der ursprünglich im unbehandelten Fluid vorhandenen Teilchen in der Sammelkammer (22); **dadurch gekennzeichnet, dass** die Teilchen und ein Teil des Basisfluids den mikroporösen Körper (32) unter dem Einfluss einer umgebenden Schwerkraft, unter Ausschluss einer anderen äußeren Kraft, durchdringen und in die Sammelkammer (22) geführt und in dieser gesammelt werden; und

wobei der mikroporöse Körper betriebsmäßig ein Fluidströmungsregime erzeugt, das eine erste Fluidströmung mit einer ersten Strömungsrate durch den Mikrokanal (16) und eine zweite Fluidströmung mit einer zweiten Strömungsrate durch die Sammelkammer (22) umfasst, und die zweite Strömungsrate einen Bruchteil der ersten Strömungsrate beträgt, und weiter einen Schritt des Vorfüllens der Vorrichtung vor dem Einleiten des unbehandelten Fluids in den Mikrokanal (16) umfassend.

2. Verfahren nach Anspruch 1, das weiter das erneute Durchleiten des Fluids durch den mikroporösen Körper (32) und das erneute Einführen in den Mikrokanal (16) umfasst.

3. Verfahren nach Anspruch 1, wobei das unbehandelte Fluid eines oder mehrere aus Vollblut, einem Zellextrakt oder einem Gewebeextrakt umfasst.

4. Verfahren nach Anspruch 1, wobei es sich bei den Teilchen um Blutzellen handelt.

5. Verfahren nach Anspruch 1, wobei das behandelte Fluid Blutplasma umfasst.

**Revendications**

1. Procédé de séparation de particules dispersées à l'intérieur d'un fluide de base et présentant une différence de densité relative par rapport au fluide de base, comprenant :

la fourniture d'un dispositif de séparation (10) comprenant :
un micro-canal (16) de longueur l et de hauteur h disposé entre une entrée de fluide (12) et une sortie de fluide (14) ; un corps microporeux (32) définissant au moins une partie du micro-canal ; et une chambre de collecte (22) sur un côté opposé du corps microporeux ; dans lequel les particules et une partie du fluide de base traversent le corps microporeux sous l'influence d'une force de gravitation ambiante, et sont introduites et collectées dans la chambre de collecte ;
l'introduction d'un échantillon de fluide non traité comprenant des particules dispersées à l'intérieur d'un fluide de base dans le micro-canal (16) via l'entrée de fluide (12) ;
la séparation d'au moins une partie des particules du fluide non traité pour fournir un courant de fluide traité au niveau de la sortie de fluide (14) ; et
la récupération d'au moins une partie des particules initialement présentes dans le fluide non traité dans la chambre de collecte (22) ; **caractérisé en ce que** les particules et une partie du fluide de base traversent le corps microporeux (32) sous l'influence d'une force de gravitation ambiante à l'exclusion d'une autre force externe, et sont entrées et collectées dans la chambre de collecte (22) ; et
dans lequel le corps microporeux génère de façon fonctionnelle un régime de flux de fluide comprenant un premier flux de fluide présentant un premier débit à travers le micro-canal (16) et un second flux de fluide présentant un second débit à travers la chambre de collecte (22) et le second débit est une fraction du premier débit, et comprenant en outre une étape d'amorçage du dispositif avant introduction du fluide non traité dans le micro-canal (16).

2. Procédé selon la revendication 1, comprenant en outre le fait que le fluide retraverse le corps microporeux (32) et entre à nouveau dans le micro-canal (16).

3. Procédé selon la revendication 1, dans lequel le fluide non traité comprend un ou plusieurs de sang total, d'un extrait cellulaire ou d'un extrait tissulaire.

4. Procédé selon la revendication 1, dans lequel les particules sont des cellules sanguines.

5. Procédé selon la revendication 1, dans lequel le fluide traité comprend du plasma sanguin.

FIG. 1B

FIG. 1C

FIG. 1A

FIG. 2

FIG. 3

FIG. 4

FIG. 5

FIG. 6

FIG. 7A

FIG. 7B

FIG. 8A

FIG. 8B

FIG. 8C

FIG. 8D

FIG. 9

FIG. 10

FIG. 11A

FIG. 11B

FIG. 12

EP 3 083 051 B1

FIG. 13

FIG. 14

**EP 3 083 051 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012149021 A1
- US 2005148064 A1